# EUROPEAN PATENT SPECIFICATION

(11) **EP 0 519 084 B1**
(45) Date of publication and mention of the grant of the patent: **23.10.1996**
(21) Application number: 92901915.6
(22) Date of filing: 25.12.1991
(51) Int. Cl.: C07C 27/10, C07C 37/58, C07C 37/60, C07C 39/04, C07C 45/34, C07C 49/08, B01J 23/40, C07C 45/35, C07C 45/36, C07C 45/28

(54) **PROCESS FOR PRODUCING SIMULTANEOUSLY AN AROMATIC HYDROXY COMPOUND AND A CARBONYL COMPOUND**
VERFAHREN ZUR GLEICHZEITIGEN HERSTELLUNG EINER AROMATISCHEN HYDROXYVERBINDUNG UND EINER CARBONYLVERBINDUNG
PROCEDE DE PRODUCTION SIMULTANEE D'UN COMPOSE HYDROXY AROMATIQUE ET D'UN COMPOSE CARBONYLE

(30) Priority: 26.12.1990 JP 406712/90; 12.03.1991 JP 46558/91
(43) Date of publication of application: 23.12.1992
(73) Proprietor: MITSUI TOATSU CHEMICALS, Inc., Chiyoda-Ku Tokyo 100 (JP)
(72) Inventor: MATSUDA, Fujio, Kamakura-shi, Kanagawa 248 (JP); MIYATA, Katsuharu, Yokohama-shi, Kanagawa 247 (JP); KATOH, Takazo, Ashigarakami-gun, Kanagawa 258 (JP); SUGIYAMA, Eiiti, Odawara-shi, Kanagawa 256 (JP); INOUE, Kaoru, Totsuka-ku, Yokohama-shi, Kanagawa 244 (JP)
(74) Representative: Nicholls, Kathryn Margaret
(86) International application number: JP9101754
(87) International publication number: WO9212114

(56) References cited:
- BE-A- 679 339
- FR-A- 2 310 987
- JP-A-50 142 518
- JP-A-56 087 527
- JP-A-61 236 738
- JP-A-62 067 038
- JP-A-62 292 738
- JP-B-45 036 495
- JP-B-45 038 044
- US-A- 2 439 812

## Description

The present invention relates to a method for simultaneously preparing a carbonyl compound and an aromatic hydroxy compound such as phenols, which are quite important as industrial starting materials and in particular, to a novel method for directly preparing phenols, in high yields, from aromatic compounds such as benzene and oxygen under mild reaction conditions.

### [Background Art]

Up to now, there have been known methods comprising reacting benzenes with oxygen in a gas or liquid phase in the presence of a catalyst, as means for directly preparing, through a single step, aromatic hydroxy compounds such as phenols from aromatic compounds such as benzene and oxygen. For instance, benzene is completely oxidized in a gas phase reaction of benzene and oxygen as disclosed in Japanese Unexamined Patent Publication (J.P. KOKAI) No. Sho 56-87527 and the selectivity to phenol is very low. Moreover, as liquid phase reactions, there have been known, for instance, methods for oxidizing benzene with a copper salt and oxygen as disclosed in YUKI GOSEI KAGAKU SHI (Journal of Organic Synthesis Chemistry), 1983, 41, p. 839. In these methods, however, the conversion of benzene is extremely low and the yield of phenol is correspondingly low.

Furthermore, J.P. KOKAI No. Hei 2-19809 discloses a method for oxidizing benzene with oxygen in the presence of 1,10-phenanthroline and carbon monooxide using a palladium catalyst, but the yield of phenol is likewise very low in this method. For this reason, there has been desired the development of a method for preparing phenol through direct oxidation of benzene, but such a method has not yet been industrialized.

In addition, J.P. KOKAI No. Hei 2-236338 discloses a method for preparing phenol through the reaction of benzene with water in the presence of a metal phosphate catalyst. According to this method, phenol can be obtained in a high yield on the order of about 6% which has not conventionally been achieved, but the reaction requires the use of a high temperature on the order of about 500°C and, therefore, this method is still insufficient from the economical standpoint.

On the other hand, the so-called Wacker method has been known as an optimum industrial method for preparing carbonyl compounds such as ketones and aldehydes from olefins. More specifically, this method comprises reacting olefins, water and oxygen in the presence of palladium ions and copper ions in a liquid phase to give carbonyl compounds such as aldehydes and ketones.

In this method, acetaldehyde is produced if ethylene is used as a starting olefin and acetone is obtained if propylene is used as a starting olefin. Carbonyl compounds obtained from other olefins are detailed in Angew. Chem., 1959 Vol. 71, p. 176.

US-A-2439812 discloses the use of olefins as "inductors" in the oxidation of benzene in the absence of catalyst, under pressure and at elevated temperature.

However, there has not yet been known any method for simultaneously preparing carbonyl compounds and aromatic hydroxy compounds such as phenols. Besides, there has not yet been known any method for preparing phenol through direct oxidation of benzene under a mild temperature condition in the order of not more than 300 °C and in an extremely high yield of the order of about 10%.

### [Disclosure of Invention]

An object of the present invention is to provide a method for preparing aromatic hydroxy compounds from aromatic compounds such as benzene and derivatives thereof under mild reaction conditions in a high yield. Another object of the present invention is to provide a method for preparing aromatic hydroxy compounds which can further improve the yield of the aromatic hydroxy compounds and simultaneously prepare carbonyl compounds by carrying out the reaction in the presence of olefins in addition to aromatic compounds.

The inventors of this invention have conducted intensive studies to accomplish the foregoing objects and as a result, have developed a method for preparing aromatic hydroxy compounds such as phenol, in a high yield, from aromatic compounds such as benzene and derivatives thereof and oxidizing agents such as oxygen under mild reaction conditions. The method of the present invention comprises the step of directly oxidizing aromatic compounds with an oxidizing agent to give aromatic hydroxy compounds wherein the oxidation reaction is carried out in the presence of a catalyst comprising at least one member selected from metals of the platinum group or compounds of the platinum group metal and at least one member selected from metals capable of forming metal compounds in which the metal has at least two kinds of valencies or compounds thereof and wherein the reaction is carried out in the coexistence of olefins represented by ethylene, propylene, butene-1, butadiene, acrylonitrile, acrylic acid and styrene and others.

It is essential in the method of the present invention to make olefins coexist in the reaction system, whereby the yield of the aromatic hydroxy compound is substantially improved. In addition, the method can simultaneously provide carbonyl compounds derived from the olefins, which are represented by acetaldehyde, acetic acid, acetone, acrolein, methyl ethyl ketone, α -ketoester and α -ketocarbonyl compounds and others. According to the present invention, there is thus provided a method for simultaneously preparing aromatic hydroxy compounds and carbonyl compounds, which has an extremely high efficiency and is quite advantageous from the economical standpoint.

### [Best Mode for Carrying Out the Invention]

Examples of the aromatic compounds used in the method of this invention include 6-membered carbon ring condensed compounds such as benzene, substituted benzenes, naphthalene, substituted naphthalenes, anthracene, substituted anthracene, durene and substituted durene; and hetero aromatic compounds such as thiophene, substituted thiophene, furan, substituted furan, pyridine, substituted pyridines, quinoline and substituted quinolines. Examples of substituents of these aromatic compounds include alkyl groups such as methyl, ethyl, propyl and butyl groups; carboxyl group, nitro group, amino groups, cyano group, acetyl group, alkoxy groups, hydroxyl group, acetoxy group, halogen atoms, mercapto groups and thioalkoxy groups. These aromatic compounds are used alone or in any combination of two or more of them.

The olefins used in the method of this invention are aliphatic and alicyclic hydrocarbons each having at least one carbon-carbon double bond in the molecule and preferably those having up to 30 carbon atoms. These olefins may be hetero atom-containing compounds such as oxygen atom-containing, nitrogen atom-containing and sulfur atom-containing compounds. The olefins also include those partially substituted with aromatic groups. If the olefin has at least two carbon-carbon double bonds, these double bonds may be conjugated one, but it is preferred that at least one double bond be non-conjugated one. Specific examples of the olefins are aliphatic hydrocarbons such as ethylene, propylene, butenes, butadienes, pentenes, pentadienes, pentatrienes, hexenes, hexadienes and hexatrienes; oxygen atom-containing olefins such as vinyl alcohol, allyl alcohol, acrylic acid, acrylates, vinyl ethers and allyl ethers; nitrogen atom-containing olefins such as allyl amines; sulfur atom-containing olefins such as allyl mercaptan and vinyl thioethers; alicyclic olefins such as cyclopentene and cyclopentenes carrying substituents, cyclopentadienes and cyclopentadienes carrying substituents, cyclohexene and cyclohexenes carrying substituents, cyclohexadienes and cyclohexadienes carrying substituents, cyclooctene and cyclooctenes carrying substituents, cyclooctadienes and cyclooctadienes carrying substituents, and cyclooctatrienes and cyclooctatrienes carrying substituents; and aromatic olefins such as styrene, substituted styrenes, divinylbenzene, substituted divinylbenzenes, propenylbenzene, substituted propenylbenzene_{,} butenylbenzene, substituted butenylbenzenes, cyclohexenylbenzene, substituted cyclohexenylbenzenes, vinylnaphthalene and substituted vinylnaphthalenes. Examples of substituents of the foregoing alicyclic and aromatic olefins are alkyl groups such as methyl, ethyl and propyl groups; carboxyl group, nitro group, amino groups, cyano group, acetyl group, hydroxyl group, acetoxy group, alkoxy groups; halogen atoms such as chlorine, bromine and fluorine atoms; mercapto groups and thioalkoxy groups. These olefins may be used alone or in combination.

The "aromatic hydroxy compound" produced according to the method of the present invention is defined to be a compound in which at least one hydroxyl group is directly bonded to the carbon atom which is a member of the aromatic ring of the foregoing aromatic compound and more specifically_{,} a compound in which at least one of carbon-hydrogen bonds on the carbon atoms which are members of the aromatic ring of the foregoing aromatic compound is replaced with at least one carbon-hydroxyl bond. For instance, phenol, catechol hydroquinone and resorcin are, for instance, formed from benzene; naphthols and dihydroxynaphthalenes are, for instance_{,} formed from naphthalene; mono- and poly-hydroxytoluenes are, for instance, formed from toluene; and methoxyphenol and methoxyresorcin are, for instance, formed from anisole. Compounds having at least one hydroxyl group directly bonded to the aromatic carbon are likewise formed from the foregoing aromatic compounds. A quinone such as benzoquinone, naphthoquinone or anthraquinone is formed simultaneous with the aromatic hydroxy compounds depending on reaction conditions selected.

The "carbonyl compound" produced according to the method of the present invention is defined to be a compound having a carbon-oxygen double bond and more specifically a compound having a carbon-oxygen double bond derived from at least one carbon-carbon double bond of the foregoing olefin compounds. For instance, carbonyl compounds derived from ethylene include, for instance, acetaldehyde and acetic acid; carbonyl compounds derived from propylene include, for instance, acetone, acrolein and propionic acid; those derived from butenes are, for instance, methyl ethyl ketone, butyl aldehyde and butyric acid. Aldehydes, carboxylic acids and ketones are likewise derived from the foregoing olefins.

Metals of the platinum group used, as catalysts, in the method of this invention include those represented by elementary symbols: Ru, Rh, Pd, Os, Ir and Pt which are preferably used in the form of, for instance, powders or particles. These metals may be supported on carriers such as silica, alumina, active carbon and zeolite.

In addition, the compounds of metals of the platinum group include, for instance, inorganic and organic metal compounds of Ru, Rh, Pd, Os, Ir and Pt and metal salts and metal complexes thereof. Specific examples thereof are as follows (expressed in terms of molecular formulas or rational formulas):

Examples of Ru compounds include RuF₅, RuF₆, RuCl₃, RuCl₄, RuBr₃, RuI₃, RuO₂, RuO₄, Ru(OH)₃, RuS₂, K₂[RuCl₆], [Ru(NH₃)₆]Cl₂, [Ru(NH₃)₅(N₂)]Cl₂, 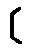 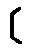 Ru(C₁₀H₈N₂)₃ 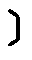 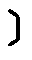 Cl₂·6 H₂O, 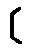 Ru(NH₃)₆ 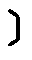 Br₃, 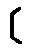 RuCl(NH₃)₅ 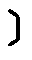 Cl₂, K₄ 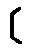 Ru(CN)₆ 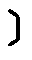 ·3H₂O, RuCl₂(CO)₃, RuCl₂{P(C₆H₅)₃}₃, RuCl(CO)₃(C₃H₅), Ru(CO)₂(C₃H₅)₂, Ru(CH₃)(CO)₂(C₅H₅)₂, Ru₂(CO)₄(C₅H₅)₂, Ru₃(CO)₁₂, Ru(C₅H₅)₂ and Ru(C₅H₅)(C₅H₄COCH₃).

Examples of Rh compounds include RhF₃, RhF₅, RhF₆, RhCl_{3,} RhBr₃, Rh₂O₃, RhO₂, Rh₂S₃, Rh₂(SO₄)₃, Rh(NO₃)₃, KRh(SO₄)₂, Na₃[RhCl₆], K₃[RhCl₆], (NH₄)₃[RhCl₆], [Rh(C₅H₇O₂)₃], Rh₂(CH₃COO)₄(H₂O)₂, Na₃[Rh(NO₂)₆], Rh[NH₃)₆]Cl₃, [RhCl(NH₃)₅]Cl₂, [Rh(C₂H₈N₂)₃]Cl₃·3H₂O, K₃[Ru(CN)₆], RhCl{P()₃}₃, RhCl(CO){P(C₆H₅)₃}₂, RhH(CO){P(C₆H₅)₃}₃, Rh₂Cl₂(CO)₄, Rh₄(CO)₁₂, Rh₄(CO)₁₆, Rh(C₂H₄)₂(C₅H₅), Rh{C₄(C₆H₅)₄}(C₅H₅), Rh₂Cl₂(C₂H₄)₄, [Rh(C₅H₅)₂][PF₆], [Rh(C₅H₅)₂Br₃ and Rh₂Cl₂(C₈H₁₂)₂.

Examples of Pd compounds include PdF₂, PdCl₂, PdBr₂, PdI₂, PdO, PdO₂, PdS, PdSO₄, Pd(NO₃)₂, Pd(CN)₂, Na₂[PdCl₄], K₂[PdCl₄], (NH₄)₂[PdCl₄], K₂[PdCl₆], (NH₄)₂[PdCl₆], K₂[PdBr₄], Pd(CH₃COO)₂, Pd(C₅H₇O₂), [Pd(NH₃)₄]Cl₂, [Pd(C₂H₈N₂)₂]Cl₂, [PdCl₂(C₂H₈CN)₂], [PdCl₂(C₆H₅CN)₂], K₂[Pd(CN)₄], Pd{P(C₆H₅)₃}₄, PdCl₂{P(C₆H₅)₃}₂, Pd(C₄H₉NC)₂, Pd(O₂)(C₄H₉NC)₂, Pd₂(C₁₇H₁₄O)₃, Pd(CH₃)₂(C₁₀H₈N₂), Pd(CH₃)₂{P(C₂H₅)₃}₂, Pd₂Cl₄(C₂H₄)₂, Pd(C₂H₄){P(C₆H₅)₃}₂, Pd(C₃H₅)_{2,} Pd₂Cl₂(C₃H₅)₂, Pd(C₃H₅)(C₅H₅), PdCl₂{C₄(C₆H₅)₄}, PdCl₂(C₈H₁₂) and {Pd(C₅H₇O₂)(C₈H₁₂)}BF₄.

Examples of Os compounds include OsF₄, OsF₅, OsF₆, OsCl_{3,} OsCl₄, OsBr₄, OsF₂O₃, OsO₂, OsO₄, OsS₂, Na₂[OsCl₆], K₃[OsCl₆], K₂[OsCl₆], K₂[O₂ClO₂], K₄[Os(CN)₆], O₃(CO)₁₂, Os(C₆H₅)₂ and Os(C₅H₅)(C₅H₄COCH₃).

Examples of Ir compounds include IrF₃, IrF₅, IrF₆, IrCl₂, IrCl₃, IrCl₄, IrBr₃, IrBr₄, IrI₃, IrI₄, Ir₂O₃, IrO₂, Ir₂S₃, IrS₂, IrSe₂, K₃[IrCl₆], Na₂[IrCl₆], K₂[IrCl₆], (NH₄)₂[IrCl₆], K₃[Ir(C₂O₄)₃]·4H₂O, [Ir(NH₃)₆]Cl₃, [IrCl(NH₃)₅]Cl₂, K₃[Ir(CN)₆], trans-IrCl(CO){P(C₆H₅)₂}, IrH(CO){P(C₆H₅)₃}, Ir(CO)₂(C₅H₅), IrCl{P(C₆H₅)₃}₃, trans-IrCl (N₂){P(C₆H₅)₃}₂, IrCl(C₂H₄):{P(C₆H₅)₃}, Ir₂Cl₂(C₈H₁₂)₂ and Ir₂(CO)₁₂.

Examples of Pt compounds include PtF₂, PtF₄, PtF₆, PtCl_{2,} PtCl₄, PtBr₂, PtBr₄, PtI₂, PtI₄, PtO, Pt₃O₄, PtO₂, PtS, PtS₂, Pt(SO₄)₂, Pt(CN)₂, Na₂[PtCl₄], K₂[PtCl₄], (NH₄)₂[PtCl₄], H₂[PtCl₆], Na₂[PtCl₆], K₂[PtCl₆], (NH₄)₂[PtCl₆], K₂[PtBr₄], Na₂[PtBr₆], K₂[PtBr₆], (NH₄)₂[PtBr₆], Na₂[PtI₆], K₂[PtI₆], (NH₄)₂[PtI₆], H₂[Pt(OH)₆], Na₂[Pt(OH)₆], K₂[Pt(OH)₆], [Pt(C₅H₇O₂)₂], [Pt(NH₃)₄]Cl₂, cis-[PtCl₂(NH₃)₂], trans-[PtCl₂(NH₃)₂], [Pt(NH₃)₄][PtCl₄], PtCl₂(C₆H₅CN)₂], [Pt(C₂H₈N₂)₂]Cl₂, K₂[Pt(SCN)₄], K₂[Pt(CN)₄]_{,} Ba[Pt(CN)₄], [Pt(NH₃)₆]Cl₄, [PtCl(NH₃)₅]Cl₃, cis-[PtCl₄(NH₃)₂], trans-[PtCl₄(NH₃)₂], [Pt(C₂H₈N₂)₃]Cl₄, cis-PtCl₂{P(C₆H₅)₃}₂, Pt{P(C₆H₅)₃}₄, Pt(O₂){P(C₆H₅)₃}₂, Pt(CO)₂{P(C₆H₅)₃}₂, trans-[PtI(CH₃){P(C₂H₅)₃)₂], cis-[Pt(C₆H₅)₂{P(C₂H₅)₃}₂], trans-[Pt(C₆H₅)₂{P(C₂H₅)₃}₂], cis-[PtCl₂(C₆H₅NC){P(C₂H₅)₃}], Pt{C₂C₆H₅)₂{P(C₆H₅)₃}₂], Pt(C₂H₄)₃, Pt(C₂H₄){P(C₆H₅)₃}₂], K[PtCl₃(C₂H₄)], Pt(C₃H₅)₂, Pt(C₈H₁₂)₂, PtCl₂(C₈H₁₂) and [PtI(CH₃)₃]₄.

These compounds of metals of the platinum group may have water of crystallization in the molecules and such hydrates can be used without any trouble. In addition, these compounds of metals of the platinum group can, of course, be supported on carriers such as silica, alumina, active carbon and zeolite. At least one member of these platinum group metals and compounds thereof is used in the reaction.

The amount of these platinum group metals or compounds thereof is not limited to a particular range, but if the reaction is, for instance, carried out batchwise, the amount thereof preferably ranges from 0.0005 to 10 g and more preferably 0.01 to 1 g (expressed in terms of the reduced metal weight) per 100 g of benzene.

The metal capable of forming metal compounds in which the metal may have at least two kinds of valencies and usable, as catalysts, in the method of this invention means a metal (except for those of the platinum group) capable of forming metal compounds in which the metal may have a plurality of valencies, for instance, monovalent metal compounds and divalent metal compounds. Specifically, a single such metal can form, for instance, two kinds of halides: MX and MX₂ (wherein M is a metal atom and X represents a halogen atom) as the compounds thereof, i.e., the metal forms monovalent and divalent halides. Thus, the metal means those each of which has a plurality of valencies and can form different metal compounds comprising the metal in different valence states.

More specifically, copper, for instance, can form compounds such as CuCl, CuBr, CuI, Cu₂O and Cu₂CO₃ in which the valency of copper is 1; and compounds such as CuCl₂, CuF₂, Cu(ClO₃)₂, CuSO₄ and CuO in which the valency of copper is 2. As has been illustrated above, the metal capable of forming metal compounds whose metal has at least two kinds of valencies means metals each having a plurality of valencies and metal compounds are present for each valency of a particular metal.

Specific examples of metals thus defined include those represented by the following elementary symbols: Ag, Au, Ce, Co, Cr, Cs, Cu, Eu, Fe, Ge, Hg, In, Mn, Mo, Nb, Ni, Np, Pa, Pb, Po, Pr, Rb, Re, Sb, Se, Sm, Sn, Ta, Te, Th, Tl, U, V, W, Yb and Zr. At least one of these metals or compounds thereof is used in the reaction.

Specific examples of compounds of these metals are as follows:

Examples of Ag compounds include inorganic and organic silver compounds such as Ag₂F₂, AgF, AgF₂, AgCl, AgClO₃, AgClO₄, AgBr, AgBrO₃, AgI, AgIO₃, AgIO₄, Ag₂O, AgO, Ag₂S, Ag₂SO₃, Ag₂SO₄, Ag₂S₂O₃, Ag₂Se, Ag₂Te, AgN₃, AgNO₂, AgNO₃, AgCNO, Ag[PF₆], Ag₂HPO₄, Ag₂PO₄, Ag₄P₂O₇, AgPO₃, Ag₃AsO₃, Ag₃AsO₄, Ag[SbF₆], Ag₂C₂, Ag₂CO₃, AgCN, AgNCO, AgSCN, Ag[BF₄], Ag(CH₃COO), Ag₂(C₂O₄), Ag₂CrO₄, Ag₂CrO₇, [Ag(NH₃)₂]₂SO₄, K[Ag(CN)₂], K[Ag(SCN)₂], Ag(CH₃) and Ag(C₆H₅).

Examples of Au compounds are organic and inorganic gold compounds such as AuCl, AuCl₃, AuBr, AuBr₃, AuI, AuI₃, Au₂O₃, Au(OH)₃, Au₂S, Au₂S₃, Au₂P₃, AuCN, Au(CN)₃·3H₂O, H[AuCl₄], Na[AuCl₄], K[AuCl₄], NH₄[AuCl₄], K[AuBr₄], K[AuI₄], K[Au(OH₄)], H[Au(NO)₄], K[Au(CN)₂], K[Au(CN)₄], Au(CH)₃{P(C₆H₅)₃}, Au(CH₃)₃ and AuBr(CH₃)₂.

Examples of Ce compounds are inorganic and organic cerium compounds such as CeF₃, CeF₄, CeCl₃, CeBr₃, CeI₃, Ce(ClO₄)₃, Ce₂O₃, CeO₂, Ce(OH)₃, Ce(OH)₄, Ce₂S₃, Ce₂(SO₄)₃, Ce₂(SO₄)₃, Ce₂(SO₄)₂, Ce₂Se₃, Ce₂Te₃, Ce(NO₃)₃, Ce(NO₃)₄, CePO₄, CeC₂, Ce₂(CO₃)₃, CeB₆, CeH₃, Ce(NH₄)(SO₄)₂, Ce(NH₄)₂(NO₃)₅, Ce(NH₄)₂(NO₃)₆, Ce(CH₃COO)₃, Ce₂(C₂O₄)₃, [Ce(C₅H₇O₂)₃] and [Ce(C₅H₇O₂)₄].

Examples of Co compounds include inorganic and organic cobalt compounds such as CoF₂, CoF₃, CoCl₂, Co(ClO₄)₂, CoBr₂, CoI₂, CoO, Co₂O₃, Co₃O₄, Co(OH)₂, CoS, Co₂S₃, CoS₂, CoSO₄, Co(SO)₄, CoSe, CoSeO₄, Co(NO₃)₂, Co₂P, Co₃(PO₄)₂, CoAsS, Co₃(AsO₄)₂, CoCO₃, Co(CN)₂, Co(SCN)₂, CoSi₂, Co₂SiO₄, CoCrO₄, Al₂CoO₄, CoFe₂O₄, Co(CH₂COO)₂, Co(CH₃COO)₃, Co(C₂H₄), Co(NH₄)₂(SO₄)₂, CoK₂(SO₄)₂, [Co(NH₃)₆]Cl₂, Co(C₅H₇O₂)₂, Cs[CoCl₄], Co[Hg(SCN)₄], [Co(NH₃)₆]Cl₃, [CoCl(NH₃)₅]Cl₂, [Co(NH₃)₅(H₂O)]Cl₂, [Co(NO₂)(NH₃)₅]Cl₂, cis-[CoCl₂(NH₃)₄]Cl, trans-CoCl₂(NH₃)₄]Cl, cis-[Co(NO₂)₂(NH₃)₄]Cl, trans-[Co(NO₂)₂(NH₃)₄]Cl, [CoCO₃(NH₃)₄]Cl, mer-[Co(NO₂)₃(NH₃)₃], trans-NH₄[Co(NO₂)₄[Co(NH₃)₂], Na₃[Co(NO₂)₆], K₃[Co(NO₂)₆], [Co(C₂H₈N₂)₃]Cl₃, [CoCl₂(C₂H₈O₂)₂]Cl, Na₃[CoCO₃)₃], K₃[Co(C₂O₄)₃, [Co(C₅H₇O₂)₃], K₃[CoF₆], K₃[Co(CN)₆], K[Co(C₁₀H₁₂N₂O₈], CoCl{P(C₆H₅)₃}₃, CoCl₂{P(C₆H₅)₃}₂, CoH(CO)₄, Co(C₃H₅)(CO₃), Co(CO)₃(NO), Co(CO)₂(C₅H₅), Co₂(CO)₈, Co₂(CO)₆(C₂H₂), Co₃(CO)₉(CH), Co₄(CO)₁₂, Co(C₅H₅)₂, [Co(C₆H₅)₂]⁺Br₃, Co(C₅H)(C₄H₄), Co(C₈H₁₂)(C₅H₅) and Co(CH₃)₂{P(C₆H₅)₃}(C₅H₅).

Examples of Cr compounds include inorganic and organic chromium compounds such as CrF₂, CrF₃, CrCl₂, CrCl₃, Cr(CI₄)₃, CrBr₃, CrI₂, CrI₃, CrI₂O₂, CrO, Cr₂O₃, CrO₃, Cr(OH)₂, Cr(OH)₃, CrS, Cr₂S₃, CrSO₄, Cr₂(SO₄)₃, CrN, Cr(NO₃)₃, CrP, CrPO₄, Cr₃C₂, Cr₃Si₂, CrB, CrB₂, Cr(CH₃COO)₂, Cr(CH₃COO)₃, Cr₂(C₂O₄)₃, CrK(SO₄)₂, CsCr(SO₄)₂, Cr(NH₄)(SO₄)₂, [Cr(H₂O)₆]Cl₃, [Cr(C₅H₇O₂)₃], K₃[Cr(C₂O₄)₃], [Cr(NH₃)₆]Cl₃, [CrCl(NH₃)₅]Cl₂, [Cr(NH₃)₅(H₂O)]Cl₃, [Cr(C₂H₈N₂)₃]Cl₃, CrCl₂(C₂H₈N₂)₂]Cl, CrCl₂(C₂H₈N₂)₂ 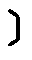 Cl, K₃[Cr(NCS)₆], NH₄[Cr(NCS)₄(NH₃)₂], K₃[Cr(CN)₆], Cr(CO)₆, Cr(CO)₅{P(C₆H₅)₃, Cr(CO)₃(C₆H₆), CrH(CO)₃(C₅H₅), Cr₂(CO)₆(C₅H₅)₂, Cr(C₆H₆)₂, Cr(C₅H₅)₂, Cr(C₃H₅)₃ and Cr(CH₃)₄.

Examples of Cs compounds include inorganic and organic cesium compounds such as CsF, CsCl, CsClO₃, CsClO₄, CsBr, CsBr₃, CsI, CsI₃, Cs₂O, Cs₂O₂, CsO₂, Cs₂O₃, CsOH, Cs₂S, Cs₂S₂, Cs₂S₃, Cs₂SO₄, CsHSO₄, CsN₃, CsNO₃, Cs₂CO₃, CsHCO₃, CsCN, CsSCN, CsBF₄, CsH, Cs[BH₄], Cs(CH₃COO), Cs₂(C₂O₄), Cs[ICl₂], Cs[IBr₂], Cs[BrCl₂], AlCs(SO₄)₂, CsTi(SO₄)₄, CrCs(SO₄)₂ and Cs₂[CoCl₄].

Examples of Cu compounds include inorganic and organic copper compounds such as CuF₂, CuCl, CuCl₂, Cu(ClO₃)₂, Cu(ClO₄)₂, CuBr, CuBr₂, CuI, Cu₂O, CuO, Cu(OH)₂, Cu₂S, CuS, CuSO₄, Cu₂Se, CuSe, CuSeO₄, Cu₃N, CuN₃, Cu(N₃)₂, Cu(NO₃)₂, Cu₃P, Cu₃(PO₄)₂, Cu₃As, Cu₃(AsO₄)₂, Cu₂C₂, CuCO₃, Cu₂(CO₃)(OH)₂, CuCN, Cu(CN₂)₂, CuSCN, CuFe₂O₄, CuSiF₆, Cu[BF₄]₂, Cu(CH₃COO)₂, Cu(C₂O₄), [Cu(C₅H₇O₂)₂], [Cu(NH₂CH₂COO)₂], [Cu(NH₃)₄]SO₄, [Cu(C₂H₈N₂)]SO₄, K₃[Cu(CN)₄], LiCu(CH₃)₂, Cu(C₆H₅), Cu(C≡CC₆H₅) and Cu(C₅H₅){P(C₂H₅)₃}.

Examples of Eu compounds include inorganic and organic europium compounds such as EuF₂, EuF₃, EuCl₂, EuCl₃, Eu(ClO₄)₃, EuBr₂, EuBr₃, EuI₂, EuI₃, EuO, Eu₂O₃, EuSO₄, Eu₂(SO₄)₃, Eu(NO₃)₃, Eu(CH₃COO)₃, [Eu(C₅H₇O₂)₃] and [Eu(C₁₁H₁₉O₂)₃].

Examples of Fe compounds include inorganic and organic iron compounds such as FeF₂, FeF₃, FeCl₂, FeCl₃, Fe(ClO₄)₂, Fe(ClO₄)₃, FeBr₂, FeBr₃, FeI₂, FeO, Fe₂O₃, Fe₃O₄, Fe₃(OH)₂, FeO(OH), FeS, Fe₂S₃, FeS₂, FeSO₄, Fe₂(SO₄)₃, FeSe, Fe₂N, Fe(NO₃)₂, Fe(NO₃)₃, Fe₂P, Fe₃P, Fe₃(PO₄)₂, FePO₄, FeAs, FeAs₂, FeAsO₄, Fe₃C, FeCO₃, Fe(SCN)₂, Fe(SCN)₃, FeSi, Fe(CH₃COO)₂, Fe(C₂O₄), Fe(C₂O₄)₃, FeMgO₄, FeMnO₄, CoFe₂O₄, Fe₂NiO₄, Fe₂ZnO₄, ZnFe₂O₄, CdFe₂O₄, Fe(NH₄)₂(SO₆)₂, FeK(SO₄)₂, Fe(C₅H₅)(CH₃COC₅H₄), Fe(C₅H₅)(C₅H₄CHO) and Fe(C₅H₅)(CH₂=CHC₅H₄).

Examples of Ge compounds include inorganic and organic germanium compounds such as GeF₂, GeF₄, GeCl₂, GeCl₄, GeBr₃, GeBr₄, GeI₂, GeI₄, GeCl₂O, GeO, GeO₂, GeS, GeS₂, GeSe₂, GeH₄, K₂[GeF₆], K₂[Ge(C₂O₄)₃], [Ge(C₅H₇O₂)₃]ClO₄, Ge(CH₃)₄, Ge(C₂H₅)₄ and Ge(C₆H₅)₄.

Examples of Hg compounds include inorganic and organic mercury compounds such as Hg₂F₂, HgF₂, Hg₂Cl₂, HgCl₃, Hg(ClO₄)₂, Hg₂Br₂, HgBr₂, Hg₂I₂, HgI₂, Hg₄ClO₃, HgO, Hg₂S, HgS, Hg₂SO₄, HgSO₄, HgSe, HgTe, Hg₃N₂, Hg₂(N₃)₂, Hg₂(NO₃)₂, Hg(NO₃)₂, HgClNH₂, Hg₃PO₄, Hg₃(PO₄)₂, Hg(CN)₂, Hg(CN)₂·HgO, Hg(CNO)₂, HgCO₃, Hg₂(SCN)₂, Hg(SCN)₂, HgCrO₄, Hg₂(CH₃COO)₂, Hg(CH₃COO)₂, KHgI₃, [HgCl₂(NH₃)₂], K₂[Hg(CN)₄], K₂[Hg(SCN)₄], CoHg(SCN)₄, Hg(CH₃)₂, Hg(CH₃Br)₂, Hg(C₃H₅)₂, Hg(C₆H₅)₂, Hg(CCl₂Br)(C₆H₅), Hg(C≡C₆H₅) 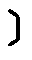 ₂, HgCl(C₆H₅), HgBr(C₆H₅), HgI(CH₃), HgI(CH₂I), Hg(CH₃COO)(C₆H₅) and Hg(CH₃COO)(CH₂C₆H₅).

Examples of In compounds include inorganic and organic indium compounds such as InF₃, InCl, InCl₃, In(ClO₄)₃, InBr, InBr₃, InI, InI₃, In₂O, InO, In₂O₃, In(OH)₃, InS₃, In₂(SO₄)₃, In₂Se₃, In₂Te₃, In(NO₃)₃, InP, InAs, InSb, In₂MgO₄, In(NH₄)(SO₄)₂, (NH₄)₃ [InF₆], (NH₄)₂[InCl₅(H₂O)], In(C₅H₅), In(CH₃)₃, In(C₅H₅)₃ and In(C₆H₅)₃.

Examples of Mn compounds include inorganic and organic manganese compounds such as MnF₂, MnF₃, MnCl₂, Mn(ClO₄)₂, MnBr₂, MnI₂, MnO, Mn₂O₃, MnO₂, Mn₃O₄, Mn₂O₇, MnO(OH), Mn(OH)₂, MnS, MnS₂, MnSO₃, MnSO₄, Mn₂(SO₄)₃, MnSe, MnTe, Mn(NO₃)₂, MnP, Mn(PH₂O₂)₂, Mn(H₂PO₄)₂, MnHPO₄, Mn₃(PO₄)₂, MnPO₄, Mn₂P₂O₇, MnAs, Mn₃C, MnCO₃, MnSi, MnSiO₃, Fe₂MnO₄, Mn(CH₃COO)₂, Mn(C₂O₄), Mn(NH₄)₂(SO₄)₂, KMn(SO₄)₂, K₂[MnF₆], [Mn(C₅H₇O₂)₂], [Mn(C₅H₇O₃), K₄[Mn(CN)₆]·3H₂O, K₃[Mn(CN)₆], Mn₂(CO)₁₀, MnBr(CO)₅, Mn(CH₃)(CO)₅, Mn(COCH₃)(CO)₅, MnH(CO)₅, Mn(CO)₄(C₃H₅), Mn(CO)₃(C₅H₅), Mn(N₂)(CO)₂(C₅H₅) and Mn(C₅H₅)₂.

Examples of Mo compounds include inorganic and organic molybdemum compounds such as MoF₃, MoF₅, MoF₆, MoCl₂, MoCl₃, MoCl₄, MoCl₅, MoBr₂, MoBr₃, MoBr₄, MoI₂, MoCl₃O, Mo₂Cl₅O₃, MoCl₄O, MoCl₂O₂, MoO₂, MoO₃, MoO(OH)₃, MoS₂, MoS₃, MoS₄, MoSe₂, MoTe₂, Mo₂C, MoC, MoSi₂, Mo₂B, MoB, MoB₂ and Mo₂(CH₃COO)₄.

Examples of Nb compounds include inorganic and organic niobium compounds such as NbF₅, NbCl₅, NbCl₃O, NbBr₅, NbI₅, NbO, NbO₂, NbO₅, NbN, NbC, NbB₂, NbH, Nb(HC₂O₄)₅, K₂[NbF₇], K₂[NbF₇], K₂[NbF₅O], Na[Nb(CO)₆], Nb(CO)₄(C₅H₅), NbCl₂(CH₃)₃ and NbCl₂(C₅H₅)₂.

Examples of Ni compounds include inorganic and organic nickel compounds such as NiF₂, NiCl₂, Ni(ClO₄)₂, NiBr₂, NiI₂, NiO, Ni(OH)₂, NiS, NiS₄, NiSO₄, NiSe, NiSeO₄, Ni(NO₃)₂, Ni₂P, Ni₂(PO₄)₂, NiAs, NiCO₃, Ni(CO₃)₂(OH)₆, Ni(CN)₂, Ni(SCN)₂, NiB, Ni[BF₄]₂, Ni(CH₃COO)₂, NiC₂H₄, NH₄NiCl₃, K₂Ni(SO₄)₂, (NH₄)₂Ni(SO₄)₂, NiFe₂O₄, K₂[NiF₄], K₂[NiF₆], [Ni(NH₃)₆]Cl₂, [Ni(C₂H₈N₂)₃]Cl₂, K₂[Ni(CN)₄], [Ni(C₃H₇O₂)₂], [Ni(C₅H₇O₂)₂(H₂O)₂], [Ni(C₂H₅)₂, (C₁₀H₈N₂), Ni{P(C₆H₅)₃}₄, 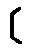 Ni(C₂H₄){P(C₆H₅)₃}₂], NiCl₂{P(C₆H₅)₃}, NiCl₂{(PC₆H₅)₃}, Ni(CO)₄, Ni(CO)₂{P(C₆H₅)₃}₂, NiCl(C₆H₅){P(C₆H₅)₃}₂, Ni(CO)₄, Ni(CO)₂(C₅H₅)₂, Ni(C₄H₉NC)₂, Ni(C₄H₉NC)₄, Ni(C₃H₅)₂, [NiBr(C₃H₅)₂], Ni(C₅H₅)₂, Ni(C₃H₅)(C₅H₅), Ni₂(C₂H₂)(C₅H₅)₂ and NiCl(C₅H₅){P(C₆H₅)₃}.

Examples of Np compounds include inorganic and organic neptunium compounds such as NpF₃, NpF₄, NpCl₃, NpCl₄, NpBr₃, NpI₃, NpO₂, Np₃O₈, NpH₂ and NpH₃.

Examples of Pa compounds include inorganic and organic protactinium compounds such as PaF₄, PaF₅, PaCl₄, PaCl₅, PaBr₅, PaI₄, PaI₅, PaO₂ and Pa₂O₅.

Examples of Pb compounds include inorganic and organic lead compounds such as PbF₂, PbF₄, PbCl₂, PbCl₄, Pb(ClO₄)₂, PbBr₂, PbI₂, PbO, Pb₃O₄, Pb₂O₃, PbO₂, Pb₃O₂(OH)₂, PbCl₂·PbO, PbS, PbSO₃, PbSO₄, Pb(HSO₄)₂, Pb(SO₄)₂, Pb₂O(SO₄), PbSe, PbSeO₄, PbTe, Pb(N₃)₂, Pb(NO₃)₂, Pb(PH₂O₂)₂, PbPHO₃ and PbHPO₄.

Examples of Po compounds include inorganic and organic polonium compounds such as PoCl₂, PoCl₄, PoBr₂, PoI₄, PoO₂ and Po(SO₄)₂.

Examples of Pr compounds include inorganic and organic praseodymium compounds such as PrF₃, PrCl₃, PrBr₃, PrI₃, Pr₂O₃, PrO₂, Pr₂S₃, Pr₂(SO₄)₃, Pr(NO₃)₃, PrC₂, Pr₂(CO₃)₃, Pr(CH₃COO)₃, Pr₂(C₂O₄)₃, [Pr(C₅H₇O₂)₃] and NHPr(SO₄)₂.

Examples of Rb compounds include inorganic and organic rubidium compounds such as RbF, RbCl, RbClO₃, RbClO₄, RbBr, RbBr₃, RbI, RbI₃, RbIO₃, Rb₂O, Rb₂O₂, RbO₂, Rb₂O₃, Rb₂O₃, RbOH, Rb₂S, RbSO₄, RbNO₃, Rb₂CO₃, Rb[BF₄], RbH, RbCH₃COO, Rb[ICl₂] and AlRb(SO₄)₂.

Examples of Re compounds include inorganic and organic rhenium compounds such as ReF₄, ReF₆, ReF₇, ReCl₃, ReCl₄, ReCl₅, ReBr₃, ReCl₄O, ReClO₃, ReO₂, ReO₃, Re₂O₇, ReS₂, Re₂S₇, K₂[ReCl₆], K₂[ReCl₅O], Re₂(CO)₁₀, ReCl(CO)₅, Re₂(CH)(CO)₅, ReH(CO)₅, Re(CO)₃(C₅H₅), Re(N₂)(CO)(C₅H₅), ReH(C₅H₅)₂ and Na[ReH₉].

Examples of Sb compounds include inorganic and organic antimony compounds such as SbF₃, SbF₅, SbCl₃, SbCl₅, SbBr₃, SbI₃, SbIO, Sb₂O₃, Sb₂O₅, SbO₂(Sb₂O₄), Sb₂O₂SO₄, Sb₂S₃, Sb₂S₅, Sb₂(SO₄)₃, Sb₂Se₃, Sb₂Te₃, SbH₃, (NH₄)₂ [SbF₅], H[SbF₆], K[SbF₆], Ag[SbF₆], K₂[Sb₂(C₄H₂O₆)₂]·3H₂O, GaSb, Sb(CH₃)₃, Sb(C₂H₅)₃ and Sb(C₆H₅)₃.

Examples of Se compounds include inorganic and organic selenium compounds such as SeF₄, SeF₆, Se₂Cl₂, SeCl₄, Se₂Br₂, SeBr₄, SeI₄, SeOF₃, SeOCl₂, SeOBr₂, SeO₂, SeO₃, SeS, SeS₂, SeSO₃, Se₄N₄, H₂Se, H₂SeO₃ and H₂SeO₄.

Examples of Sm compounds include inorganic and organic samarium compounds such as SmF₂, SmF₃, SmCl₂, SmCl₃, SmBr₂, SmBr₃, SmI₂, SmI₃, SmO₃, Sm₂S₃, Sm₂(SO₄)₃, Sm(NO₃)₃, Sm(CH₃COO)₃, [Sm(C₅H₇C₂)₃]₇ and [Sm(C₅H₅)₃].

Examples of Sn compounds include inorganic and organic tin compounds such as SnF₂, SnF₄, SnCl₂, SnCl₄, SnBr₂, SnBr₄, SnI₂, SnI₄, SnO, Sn(OH)₂, SnO₂, SnS, SnS₂, SnSO₄, Sn(SO₄)₂, SnSe, SnTe, Sn(NO₃)₄, Sn(H₂PO₄)₂, SnHPO₄, SnH₄, Sn(CH₃COO)₂, Sn(C₂O₄), Na[Sn₂F₅], Na₂[SnF₆], K₂[SnF₆], K₂[SnCl₆], (NH₄)₂[SnCl₆], K₂[SnBr₆], Sn(CH₃)₄, Sn(C₂H₅)₂, SnCl(n-C₄H₉)₃, [Sn(C₂H₅)₃]₂, Sn(C₆H₅)₄, [Sn(CH₃)₃]₂O, [Sn(n-C₄H₉)₃]₂O, Sn(n-C₄H₉)₄ and SnH(n-C₄H₉)₃.

Examples of Ta compounds include inorganic and organic tantalum compounds such as TaF₅, TaCl₂, TaCl₅, TaBr₄, TaBr₅, TaI₄, TaO₂, Ta₂O₅, TaS₂, TaN, Ta₃N₅, TaC, TaB₂, K₂[TaF₇], Na₃[TaF₈], Na(Ta(CO)₆], Ta(CO)₄(C₅H₅), TaCl₂(CH₃)₃, TaCl₂(C₅H₅)₂ and TaH₃(C₅H₅)₂.

Examples of Te compounds include inorganic and organic tellurium compounds such as TeF₄, TeF₆, TeCl₂, TeCl₄, TeBr₂, TeBr₄, TeI₄, TeO₂, TeO₃, H₂Te, H₂TeO₃ and H₆TeO₆.

Examples of Th compounds include inorganic and organic thorium compounds such as ThF₄, ThCl₄, Th(ClO₄)₄, ThBr₄, ThI₄, ThO₂, ThS₂, Th(SO₄)₂, Th₃N₄, Th((NO₃)₄, Th₃(PO₄)₄, ThC₂, ThB₄, ThB₆, Th(CH₃COO)₄, Th(C₂O₄)₂, K₂[ThF₆], K₄[(C₂O₄)₄], [Th(C₅H₇O₂)₄ and Th(C₅H)₄.

Examples of Ti compounds include inorganic and organic titanium compounds such as TiF₃, TiF₄, TlCl₂, TiCl₃, TiBr₂, TiBr₃, TiBr₄, TiI₂, TiI₄, TiO, Ti₂O₃, TiO₂, H₂TiO₃, H₄TiO₄, TiCl₂O, TiS₂, Ti₂(SO₄)₃, Ti(SO₄)₂, TiOSO₄, TiSe₂, TiN, Ti(NO₃)₄, TiC, TiSi₂, TiB, TiB₂, TiH₂, Na₂[TiF₆], K₂[TiF₆], (NH₄)₂[TiF₆], CsTi(SO₄)₂, K₂[TiO(C₂H₄)₂], [TiO(C₅H₇O₂)₂], [Ti(C₅H₅)₂]₂, Ti(CO)₂(C₅H₅)₂, TiCl(C₅H₅)₂, Ti(CH₃)₂(C₅H₅)₂, TiCl₃(C₅H₅), TiCl₃(CH₃) and Ti(CH₂C₆H₅)₄.

Examples of Tl compounds include inorganic and organic thallium compounds such as TiF, TlF₃, TlCl, TlCl₃, TlCl₄, TlBr, TlBr₃, TlI, TlI₃, Tl₂O, TlO₃, TlOH, Tl₂S, Tl₂S₃, Tl₂SO₄, T₂(SO₄)₃, Tl₂Se, TlN₃, TlNO₃, Tl(NO₃)₃, Tl₃PO₄, Tl₂CO₃, TlCN, TlSCN, Tl(CH₃COO), Tl₂(C₂O₄), AlTl(SO₄)₂, K₃[TlCl₆], Tl(C₅H₇O₂), Tl(C₅H₅) and Tl(CH₃)₃.

Examples of U compounds include inorganic and organic uranium compounds such as UF₃, UF₄, UF₆, UCl₃, UCl₄, UCl₅, UCl₆, UBr₃, UBr₄, UI₄, UO₂, U₃O₈, UO₃, UO₄, UO₂Cl₂, UO₂Br₂, UO₂I₂, UO₂(ClO₄)₂, UO₂SO₄, UO₂(NO₃)₂, (UO₂)HPO₄, U₂S₃, US₂, UO₂S, U(SO₄)₂, UC, UC₂, UB₂, UH₃, UO(CH₃COO), UO₂(C₂O₄), K₂ 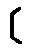 UF₆ 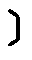 , K[UF₆], (NH₄)₂U₂O₇, K₄[U(C₂O₄)₄], [U(C₅H₇O₂)₄], [UO₂(C₅H₇O₂)₂], U(C₅O₅)₄ and U(C₈O₈)₄.

Examples of V compounds include inorganic and organic vanadium compounds such as VF₃, VF₄, VF₅, VCl₂, VCl₃, VCl₄, VBr₂, VBr₃, VI₂, VI₃, VO, V₂O₃, VO₂, O₂O₅, VF₂O, VF₃O, VClO, VCl₂O, VCl₃O, VClO₂, VBrO, VBr₂O, VBr₃O, VS, V₂S₃, V₂S₅, VSO₄, V₂(SO₄)₃, VOSO₄, VN, VC, V₂Si, VSi₂, V(CH₃COO)₃, KV(SO₄)₂, K₃[V(C₂O₄)₃], (NH₄)₂[VO(C₂O₄)₂], [V(C₅H₇O₂)₂], [VO(C₅H₇O₂)₂], Na[V(CO)₆], V(CO)₆, V(CO)₄(C₅O₅), V(C₅O₅)₂, VCl(C₅O₅)₂, V(CH₃)(C₅O₅)₂, VCl₂(C₅O₅)₂ and V(C₆O₆)₂.

Examples of W compounds include inorganic and organic tungsten compounds such as WF₆, WCl₂, WCl₄, WCl₅, WCl₆, WBr₅, WBr₆, WI₂, WI₄, WO₂, WO₃, WF₄O, WCl₄O, WBr₄O, WCl₂O₂, WS₂, WS₃, W₂C, WC, WSi₂, W₂B, WB, WB₂, W₂B₅, K₃[W₂Cl₉], K₃[W₂(CN)₈], K₄[W(CN)₈], W(CO)₆, W₂(CO)₆(C₅O₅)₂, W(CH₃)(CO)₃(C₅O₅), WCl₂(C₅H₅)₂, WH₂(C₅H₅)₂, W(CH₃)₆, Na₁₀W₁₂O₄₁, K₁₀[W₁₂O₄₂H₂], (NH₄)₁₀[W₁₂O₄₂H₂], K₆[H₂W₁₂O₄₀], Na₃[PW₁₂O₄₀], H₃[PW₁₂₀], H₄[SiW₁₂O₄₀] and K₄[SiW₁₂O₄₀].

Examples of Yb compounds include inorganic and organic ytterbium compounds such as YbF₂, YbF₃, YbCl₂, YbCl₃·6H₂O, YbBr₂, YbBr₃, YbI₂, YbI₃, YbO₃, Yb₂(SO₄)₃, Yb(NO₃)₃, Yb(CH₃COO)₃ and Yb(C₂O₄)₃.

Examples of Zr compounds include inorganic and organic zirconium compounds such as ZrF₃, ZrF₄, ZrCl₃, ZrCl₃, ZrCl₄, ZrBr₃, ZrBr₄, ZrI₃, ZrI₄, ZrO₂, ZrCl₂O, ZrS₂, Zr(SO₄)₂, ZrN, Zr(NO₃)₄, ZrO(NO₃)₂, ZrC, ZrSi₂, ZrSiO₄, ZrB₂, ZrH₂, K₂[ZrF₆], Na₂[ZrF₆], (NH₄)₃ 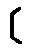 ZrF₇ 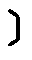 , Zr(CH₃COO)₄, [Zr(C₅H₇O₂]₄], ZrCl₂(C₅H₅)₂, (C₅ZrHCl)(C₅H₅)₂, Zr(CH₃)₂(C₅H₅)₂, ZrBr₃(C₅H₅)₂ and Zr(CH₂C₆H₅)₄.

The compounds of these metals may or may not have water of crystallization.

The amount of the metals capable of forming metal compounds in which the metal may have at least two valencies or compounds thereof is not restricted to a particular range, but if the reaction is, for instance, carried out batchwise, the amount thereof preferably ranges from 0.001 to 20 g and more preferably 0.01 to 2 g (expressed in terms of the reduced metal weight) per 100 g of benzene.

Further, it is preferred to add, to the reaction system, at least one member selected from the group consisting of elements of the iron group and compounds thereof; and alkali metals and compounds thereof, as a catalyst component. The addition thereof improves the selectivity of the reaction and the yield of phenol.

The term "alkali metals" herein means those represented by the following elementary symbols: Li, Na, K, Rb and Cs and the term "compounds of alkali metals" herein means compounds of the foregoing alkali metals. Spesific examples of the alkali metal compounds are as follows:

Li compounds such as LiF, LiCl, LiBr, LiI, LiCLO₃, LiClO₄, LiBrO₃, LiIO₃, Li₂O, Li₂O₂, LiOH, Li₂S, LiSH, Li₂SO₄, LiHSO₄, Li₃N, LiN₃, LiNO₂, LiNO₃, LiNH₂, Li₃PO₄, LiH₂PO₄, Li₃AsO₄, Li₂C₂, Li₂CO₃, LiHCO₃, LiSCN, Li(CH₃), Li(CH=CH₂), Li(i-C₃H₇), Li(n-C₄H₉), Li(s-C₄H₉), Li(t-C₄H₉), Li(C₆H₅), Li(BF₄), Li₂B₄O₇, Li[BH₄], Li[AlH₄], LiH, LiCH₃COO, Li₂(C₂O₄) and Li[Cu(CH₃)₂].

Na compounds such as NaF, NaHF₂, NaCl, NaClO, NaClO₂, NaClO₃, NaClO₄, NaBr, NaBrO₃, NaI, NaIO₃, NaIO₄, Na₃H₂IO₆, Na₂O, Na₂O, Na₂O₂, Na₂O₂·8H₂O, NaO₂, NaOH, Na₂S, NaHS, Na₂S₄, Na₂SO₃, NaHSO₃, Na₂SO₄, NaHSO₄, Na₂S₂O₃, Na₂S₂O₄, Na₂S₃O₅, Na₂S₃O₆, Na₂S₃O₇, Na₂S₃O₈, NaSO₃F, Na₂Se, Na₂SeO₃, Na₂SeO₄, Na₂Te, Na₂TeO₃, Na₂TeO₄, Na₂H₄TeO₆, NaN₃, Na₂N₂O₂, NaNO₂, NaNO₃, NaNH₂, NaPH₂O₂, NaPHO₃, NaHPHO₃, Na₃PO₄, Na₂HPO₄, Na₄P₂O₆, Na₂H₂P₂O₆, Na₄P₂O₇, Na₂H₂P₂O₇, cyclo-Na₃P₃O₉, Na₃P₃O₁₀, NaAsO₂, Na₂HAsO₃, Na₃AsO₄, NaHAsO₄, NaH₂AsO₄, NaSbO₂, Na₃SbS₄·9H₂O, BiNaO₃, Na₂C₂, Na₂CO₃, NaHCO₃, Na₂CS₃, NaCN, NaOCN, NaSCN, NaC₅H₅, Na(C₆H₅)₂CO, NaC₁₀H₁₈, Na₂SiO₃, Na₄SiO₄, Na₂GeO₃, Na₂GeO₃, Na₂B₄O₇, Na₂B₄O₇, NaBO₂, NaBO₂, NaBO₃, NaAlO₂, NaH, MgNa₂(SO₄)₂, NH₄NaHPO₄, KNaCO₃, AlNa(SO₄)₂, NaVO₃, Na₃VO₄, Na₃VO₄, Na₄V₂O₇, Na₂CrO₄, Na₂Cr₂O₇, Na₂CrO₈, Na₂MoO₄, Na₂WO₄, NaMnO₄, NaReO₄, NaFeO₂, NaRuO₄, Na₂UO₄, Na₂U₂O₇, Na₂[Sn(OH₆], Na[Sn(OH)₆], Na[XeO₆], Na[BH₄], Na[B(C₆H₅)₄], Na[BF₄], Na₃[AlF₆], Na₂[SiF₆], Na[Sn₂F₅], Na[SnF₆], NaPF₆, Na₂[TiF₆], Na₂[ZrF₆], Na₃[TaF₈], Na₃[FeF₆], Na₂[OsCl₆], Na₃[RhCl₆], Na₂[IrCl₆], Na₂[PdCl₄], Na₂[PtCl₄], Na₂[PtCl₆], Na₂[PtBr₆], Na₂[PTI₆], Na[AuCl₄], Na[V(CO)₆], Na[Nb(CO)₆], Na[Ta(CO)₆], Na[Fe(CO)₄], Na[Fe(C₂O₄)₃], Na₃[Co(CO₃)₃], Na₂[Pt(OH)₆], Na₂Ca(C₁₀H₁₂N₂O₈), Na₃[Co(NO₂)₆], Na₃[Ph(NO₂)₆], Na₄[Fe(CN)₆], Na₃[Fe(CN)₆], NH₂[Fe(CN)₅(NO)], Na₂[Fe(CN)₅(NH₃)], Na₂[Mo₂O₇], Na₂[Mo₃O₁₀], Na₆[Mo₇O₂₄], Na₄[Mo₈O₂₆], Na₃[PMo₁₂O₄₀], Na₄[SiMo₁₂O₄₀], Na₁₀[W₁₂O₄₂H₂], NaCH₃COO, NaC₂O₄, NaHC₂O₄, Na[AlH₂(OC₂H₄OCH₃)₂ and Na₂[ReH₉].

Compounds of K such as KF, KHF₂, KCl, KCLO₃, KClO₄, KBr, KBrO₃, KI, KI₃, KIO₃, KH(IO₃), KIO₄, K[BrF₄], K[BrF₆], K[ICl₂], K[ICl₄], K[Br₂], K₂O, K₂O₂, K₂O₃, KO₂, KOH, K₂S, K₂S, K₂S₂, K₂S₃, K₂S₄, K₂S₅, KHS, K₂SO₃, KHSO₃, K₂SO₄, KHSO₄, 3K₂S₂O₃, K₂S₂O₅, K₂S₂O₆, K₂S₂O₇, K₂S₂O₈, K₂S₃O₆, K₂S₄O₆, K₂S₅O₆, KSO₃F, K₂Se, K₂SeO₃, K₂SeO₄, K₂Te, K₂TeO₃, K₂TeO₄, KN₃, KNO₂, KNO₃, KNH₂, KPH₂O₂, K₂PHO₃, KHPHO₃, K₃PO₄, K₂HPO₄, KH₂PO₄, K₄P₂O₇, K₆(PO₃)₆, K₂PO₃F, KAsO₂, K₃AsO₃, K₃AsO₄, K₂HAsO₄, KH₂AsO₄, K₃AsS₃, K₃AsS₄, KSbO₃, K[Sb(OH)₆], K₃SbS₄, K₂C₂, K₂CO₃, KHCO₃, K₂C₂O₆, K₂CS₃, KCN, KOCN, KSCN, KSeCN, KC(C₆H₅)₃, KC₂(C₆H₅), K₂SiO₃, K₂Si₄O₉, K₂[sn(OH)₆], K₂SnS₃, K₂B₄O₇, K₂B₅O₈, KBO₂, K[BH₄], KAlO₂, KH, KVO₃, K₂CrO₄, K₂Cr₂O₇, K₃CrO₈, K₂MoO₄, K₂WO₄, K₂MnO₄, KMnO₄, KTcO₄, KReO₄, K₂RuO₄, KRuO₄, K₂OsO₄, K[Os(N)O₃], K₂UO₄, KCH₃COO, K₂C₂O₄, KHC₂O₄, KMgCl₃, KMgCl(SO₄), K₂Mg(SO₄)₂, K₂Mg₂(SO₄)₃, KNaCO₃, KMgH(SO₃)₂, K₂Co(SO₄)₂, K₂Ni(SO₄)₄, K₂Zn(SO₄)₂, KAl(SO₄)₂, KAl(SO₄)₂, KGa(SO₄)₂, KV(SO₄)₂, KCr(SO₄)₂, KMn(SO₄)₂, KFe(SO₄)₂, KRh(SO₄)₂, K₂[BeF₄], K[BF₄], K₂[SiF₆], K₂[GeF₆], K₂[SnF₆], K[PF₆], K[AsF₆], K[SbF₆], K₂[TiF₆], K₂[ZrF₆], K₂[NbF₇], K₂[NbF₅O], K₂[TaF₇], K₃[MoF₈], K₂[MoF₈], K₂[MnF₆], K₃[FeF₆], K₃[CoF₆], K₂[NiF₄], K₂[NiF₆], K₂[ThF₆], K₂[UF₆], K[UF₆], K₃[TlCl₆], K₂[SnCl₆], K₂[PbCl₆], K₂[SeCl₆], K₂[TeCl₆], K₃[MoCl₆], K₃[W₂Cl₉], K₃[ReCl₆], K₂[ReCl₅O], K₂[RuCl₆], K₂[OsCl₆], K₃[OsCl₆], K₂[OsCl₄O₂], K₂[RhCl₆], K₃[IrCl₆], K₂[IrCl₆], K₂[PdCl₄], K₂[PdCl₆], K₂[PtCl₄], K₂[PtCl₆], K[AuCl₄], K₂[SnBr₆], K₂[PdBr₄], K₂[PtBr₄], K₂[PtBr₆], K[AuB₄r], K₂[PtI₆], K[AuI₄], K₂[CdI₄], KHgI₃, K₃[Cr(CN)₆], K₄[Mo(CN)₈], K₄[W(CN)₈], K₃[W(CN)₈], K₄[Mn(CN)₆], K₃[Mn(CN)₆], K₄[Fe(CN)₆], K₃[Fe(CN)₆], K₂[Fe(CN)₅(NO)], KFe[Fe(CN)₆], K₄[Ru(CN)₆], K₄[Os(CN)₆], K₃[Co(CN)₆], K₃[Rh(CN)₆], K₃[Ir(CN)₆], K₂[Ni(CN)₄], K₂[Pd(CN)₄], K₂[Pt(CN)₄], K₂[Ag(CN)₄], K[Au(CN)₂], K[Au(CN)₄], K[Zn(CN)₄], K₂[Cd(CN)₄], K[Hg(CN)₄], K₃[Cr(NCS)₆], K₄[Fe(NCS)₆], K₃[Fe(NCS₆)], K₂[Pt(SCN)₄], K[Ag(SCN)₂], K[Hg(SCN)₄], K₃[Co(NO₂)₆], K₂[Pt(OH)₆], K[Au(OH)₄], K₃[Al(CO)₃], K₂[Ge(C₂O₄)₃], K₂[TiO(C₂O₄)₂], K₃[V(C₂O₄)₃], K₃[Cr(C₂O₄)₃], K₃[Fe(C₂O₄)₃], K₃[Co(C₂O₄)], K₃[Ir(C₂O₄)₃], K₄[Th(C₂O₄)₄], K₄[Th(C₂O₄)₄], K₄[U(C₂O₄)₄], K₂[ReH₉], K[Co(C₁₀H₁₂N₂O₈)], K₂[Sb₂(L·C₄H₂O₆)₂], K₆[W₁₂O₄₀H₂], K₁₀[W₁₂O₄₂H₂]and K₄[SiW₁₂O₄₀].

Compounds of Rb such as rubidium compounds listed above; and

Compounds of Cs such as cesium compounds defined above.

When the aforementioned compounds of the iron group or alkali metal compounds are used, they may or may not have water of crystallization.

Moreover, when these metals or metal compounds are practically used, these metals and metal compounds may be supported on carriers such as silica, alumina, active carbon and zeolite without any trouble upon using the same.

The amount of the metals of the iron group, compounds of metals of the iron group, alkali metals or compounds of alkali metals is not restricted to a specific range, but if the reaction is, for instance, carried out batchwise, the amount thereof preferably ranges from 0.001 to 20 g and more preferably 0.01 to 2 g (expressed in terms of the reduced metal weight) per 100 g of benzene.

The oxidizing agents used in the method of the present invention are substances capable of supplying, for instance, oxygen molecules, oxygen atoms and oxygen ions to the reaction system during the reaction.

Specific examples thereof are molecular oxygen, air, diluted air and hydrogen peroxide as well as normally used organic peroxide and inorganic peroxides which may be used alone or in combination.

In the method of the present invention, the reaction system is preferably in an acidic condition. Such an acidic normally can be established in the reaction system through addition of an acidic substance such as a Brønsted acid and a Lewis acid. Examples of Brønsted acid include mineral acids such as hydrochloric acid, sulfuric acid, nitric acid, phosphoric acid and boric acid; carboxylic acids such as acetic acid, formic acid, benzoic acid, trichloroacetic acid and oxalic acid; sulfonic acid, heteropoly- acid, zeolites and mixed oxides. Examples of Lewis acids are aluminum chloride, ferric chloride, zeolites and mixed metal oxides. The acidic substances usable in the present invention are not limited to those listed above.

Upon practicing the method of the present invention, the reaction may be carried out in a diluted condition by adding, to the reaction system, for instance, a solvent or a gas which is inert or not reactive with the catalyst and reactants. Specific examples thereof include aliphatic saturated hydrocarbons such as methane, ethane, propane, butane, hexane and cyclohexane; and inert gases such as nitrogen, argon and helium gases.

The reaction temperature is not critical in the method of this invention, but preferably ranges from 0 to 400 °C and more preferably 10 to 300°C. If the reaction temperature is extremely low, the rate of conversion of olefins and/or aromatic compounds (reactants) is low and in other words, the reaction rate is substantially lowered and as a result, the productivity of the intended product is lowered. On the other hand, if the reaction is performed at a temperature higher than 400°C, for instance, undesirable side-reactions proceed, by-products are correspondingly formed in a large amount. Further, the use of a high temperature exerts adverse influence on the stability of the starting materials, i.e., the olefins and/or aromatic compounds as well as the resulting products, i.e., carbonyl compounds and/or aromatic hydroxy compounds, the selectivity of the reaction is lowered and thus the use of such a high temperature is not preferred from the economical standpoint.

The reaction may be carried out under elevated pressure or reduced pressure or at ordinary pressure. It is not preferred to perform the reaction at an extremely low pressure from the viewpoint of the reaction efficiency (reaction efficiency per unit volume). Moreover, it is also undesirable to perform the reaction at an extremely high pressure from the viewpoint of the economy of facilities such as a reaction apparatus. In general, the pressure practically used preferably ranges from 0.05 to 300 atm. and more preferably 0.5 to 200 atm.

In addition, the reaction can be performed in a liquid phase, a gas-liquid phase or a gas phase. Further, the catalyst can be used in either a homogeneous or heterogeneous state. These catalysts can be used in the form of a solution, separated two phases, mixed two liquid phases, a slurry or as a fixed bed, a moving bed or a fluidized bed.

The present invention can be performed by a batchwise reaction, a semi-batchwise reaction in which a part of the catalyst or a starting material is continuously supplied, or a flow-through continuous reaction. Moreover, the order of addition of various components such as starting materials, acidic substances and catalysts and methods for addition thereof are not limited to specific ones.

The reaction can likewise be performed according to a semibatchwise reaction in which olefins are, for instance, added gradually over several stages depending on the consumption thereof and thus the yield of the aromatic hydroxy compound such as phenols is sometimes improved.

The reaction time (in case of flow-through reaction, the retention time or the time required for contact between the catalyst and the reactants) is not restricted to a particular range, but in general ranges from 0.1 second to 30 hours and preferably 0.5 second to 15 hours.

After completion of the reaction, the reaction product can, if necessary, be separated and recovered from other substances such as the catalyst by a method, for instance, filtration, extraction and distillation.

End products, the aromatic hydroxy compounds and carbonyl compounds as desired products can be separated and purified from the foregoing recovered reaction product by the usual separation and purification methods such as stepwise treating methods, e.g., solvent extraction, distillation, alkali-treatment, acid-treatment and appropriate combination thereof. In addition, the unreacted starting materials (the aromatic compound, olefinic compounds and acidic substances) can be recovered and recycled to the reaction system.

In case of batchwise reaction, the catalyst obtained after the recovery of the reaction products can be reused in the reaction as such or after regenerating a part or whole of the recovered catalyst.

If the reaction is carried out according to a fixed bed or fluidized bed flow-through continuous reaction method, the catalyst which is partially or entirely deactivated or whose activity is lowered can be regenerated after interrupting the reaction and then recycled, or a part of the catalyst is continuously or periodically withdrawn from the reaction system, regenerated and then reused in the reaction system. Besides, a fresh catalyst can continuously or periodically be supplied to the reactor. If the reaction is performed according to a moving bed flow-through continuous reaction method or homogeneous catalyst flow-through continuous reaction method, the catalyst can be separated and regenerated for reuse similarly to the batchwise reaction.

### [Examples]

The method of the present invention will be explained in more detail below with reference to the following Examples.

In the following Examples and Tables, the yield of the product, the hydroxy compound, and that of the carbonyl compound are calculated on the basis of the charged aromatic compound and charged olefin, respectively.

### Example 1

To a 50 ml autoclave of Hastelloy C, there were charged 4.0 g of benzene, 15 g of a 0.1 N hydrochloric acid, 0.05 g of palladium chloride and 0.05 g of copper powder, the atmosphere of the autoclave was replaced with oxygen gas and then 15 kg/cm² of oxygen gas and 15 kg/cm² of ethylene gas were pressed into the autoclave under pressure. The reaction was carried out at a temperature of 50°C for 3.0 hours with stirring, then the reaction system was cooled and the reaction solution was extracted with benzene. The resulting extract was analyzed by gas chromatography and it was confirmed that the yields of phenol, p-benzoquinone and acetaldehyde were 5.6%, 1.2% and 9.4%, respectively.

### Comparative Example 1

A reaction was performed under the same conditions used in Example 1 except that ethylene was not charged and the resulting reaction solution was analyzed in the same manner used in Example 1. As a result, the yields of phenol and p-benzoquinone were found to be 0.2% and 0.1% respectively.

This clearly indicates that the addition of an olefin to the reaction system substantially improve the yield of phenol.

### Example 2

A reaction was performed under the same conditions used in Example 1 except that 0.05 g of cupric chloride dihydrate was substituted for the copper powder used in Example 1 and the resulting product was analyzed in the same manner. As a result, the yields of phenol and acetaldehyde were found to be 1.1% and 18.3% respectively. Further, a small amount of p-benzoquinone was also detected.

### Example 3

A reaction was performed under the same conditions used in Example 2 except that 0.05 g of cuprous chloride was further added to the reaction system and the resulting product was analyzed in the same manner. As a result, the yields of phenol and acetaldehyde were found to be 2.3% and 13.6% respectively. Further, a small amount of p-benzoquinone was also detected.

### Example 4

A reaction was performed under the same conditions used in Example 2 except that 0.05g of cuprous chloride was substituted for the cupric chloride dihydrate used in Example 2 and the resulting product was analyzed in the same manner. As a result, the yields of phenol and acetaldehyde were found to be 1.8% and 10.8% respectively. Further, a small amount of p-benzoquinone was also detected.

### Example 5

A reaction was performed under the same conditions used in Example 1 except that the reaction system comprised 4.0 g of benzene, 20 g of 0.1 N sulfuric acids 0.25 g of palladium sulfate, 0.26 g of copper sulfate (pentahydrate) and 0.07 g of copper powder and that oxygen gas and ethylene gas were charged at a pressure of 5 kg/cm² . As a result, the yields of phenol and acetaldehyde were found to be 2.0% and 9.2% respectively. Almost no p-benzoquinone was detected.

### Example 6

A reaction was performed under the same conditions used in Example 1 except that the reaction system comprised 4.0 g of benzene, 6 g of acetic acid, 0.05 g of palladium acetate, 0.04 g of copper acetate and 0.9 g of water, that oxygen gas and ethylene gas were charged at a pressure of 15 kg/cm² and that the reaction was performed at 180°C for one hour and the resulting product was analyzed in the same manner. As a result, the yields of phenol and acetaldehyde were found to be 6.4% and 13.3% respectively. Further, the yield of phenyl acetate was found to be 0.7%.

### Example 7

A reaction was performed under the same conditions used in Example 1 except that 0.05 g of palladium nitrate and 0.05 g of cupric nitrate (trihydrate) were used as catalysts and that 0.1 N nitric acid was substituted for the 0.1 N hydrochloric acid used in Example 1 and the resulting product was analyzed in the same manner. As a result, the yields of phenol and acetaldehyde were found to be 5.3% and 7.1% respectively. Further, the yield of p-benzoquinone was found to be 2.1%.

### Examples 8 to 10

Reactions and analysis of the resulting products were performed in the same manner and conditions used in Example 1 except that the reaction temperature was changed to 80, 100 and 150 °C, respectively. The results obtained are summarized in Table 1. As seen from the results listed in Table 1, the yield of each reaction product was improved as the reaction temperature was raised.

**(Table 1)**

| Ex. No. | Reaction Temp.(°C ) | Yield (%) | | |
|---|---|---|---|---|
| | | Phenol | Acetaldehyde | p-Benzoquinone |
| 8 | 80 | 7.4 | 18.5 | 2.8 |
| 9 | 100 | 9.1 | 21.3 | 3.2 |
| 10 | 150 | 11.9 | 22.6 | 4.9 |

### Examples 11 to 15

Reactions and analysis of the resulting products were performed in the same manner and conditions used in Example 1 except that 4.3 g of propylene, 4.2 g of butene-1, 4.0 g of cyclopentene, 4.0 g of cyclohexene and 4.0 g of styrene were substituted for the ethylene used in Example 1. The results obtained are listed in Table 2. As seen from the data listed in Table 2, phenol and carbonyl compounds were formed in good yields.

**(Table 2)**

| Ex.No | Olefin Used | Yield (%) | | |
|---|---|---|---|---|
| | | phenol | Carbonyl Compound | p-Benzoquinone |
| 11 | propylene | 5.5 | 10.3 | 1.1 |
| 12 | butene-1 | 5.3 | 8.8 | 0.9 |
| 13 | cyclopentene | 4.9 | 7.7 | 1.0 |
| 14 | cyclohexene | 4.8 | 7.2 | 0.8 |
| 15 | styrene | 4.6 | 5.3 | 0.7 |

The carbonyl compounds obtained in these Examples are acetone (Example 11), methyl ethyl ketone (Example 12), cyclopentanone (Example 13), cyclohexanone (Example 14) and acetophenone (Example 15), respectively.

### Examples 16 to 20

Reactions and analysis of the resulting products were performed in the same manner and conditions used in Example 1 except that toluene, phenol, naphthalene, β-naphthol, anisole and p-xylene (4 g each) were substituted for the benzene used in Example 1. The results obtained are listed in Table 3. As seen from the data listed in Table 3, each product was formed in good yields.

**(Table 3)**

| Ex. | Aromatic Compound | Yield (%) | | |
|---|---|---|---|---|
| | | Hydroxy Comp. | Dihydroxy Comp. | CH₃CHO |
| 16 | toluene | 6.5 | 1.6 | 9.8 |
| 17 | phenol | (starting material) | 7.1 | 10.9 |
| 18 | naphthalene | 5.0 | 0.7 | 8.6 |
| 19 | naphthol | (starting material) | 6.0 | 7.1 |
| 20 | anisole | 5.2 | 1.4 | 9.1 |
| 21 | p-xylene | 7.0 | trace | 9.5 |

### Example 22

A reaction was performed under the same conditions used in Example 1 except that a gas mixture comprising 9% of oxygen and 91% of nitrogen was charged at 100 kg/cm² as an oxygen to be charged and it was found that the yields of phenol, p-benzoquinone and acetaldehyde were 3.9%, 0.7% and 5.6%, respectively.

### Example 23

A reaction was performed under the same conditions used in Example 22 except that the reaction time was changed to one hour. As a result, it was found that the yields of phenol_{,} p-benzoquinone and acetaldehyde were 2.4%, 0.3% and 4.1%, respectively.

### Examples 24 to 25

Reactions were performed under the same conditions used in Example 23 except that ruthenium chloride and rhodium chloride (0.28 mmole each) were respectively substituted for the palladium chloride used in Example 23. The results obtained are summarized in Table 4.

**(Table 4)**

| Ex. No. | Metal Comp. | Phenol % | p-Benzoquinone % | Acetaldehyde % |
|---|---|---|---|---|
| 24 | RhCl₃ | 1.9 | 0.2 | 3.2 |
| 25 | RhCl₃ | 1.3 | 0.1 | 2.2 |

### Examples 26 to 29

Reactions were performed under the same conditions used in Example 23 except that CoCl₂, CrCl₃, SnCl₂ and MnCl₂ (0.787 mmole each) were respectively substituted for the copper powder used in Example 23. The results obtained are summarized in Table 5. As seen from the data listed in Table 5, it is confirmed that metals other than copper also had catalytic effects.

**(Table 5)**

| Ex. No. | Metal Comp. | Phenol % | p-Benzoquinone % | Acetaldehyde % |
|---|---|---|---|---|
| 26 | CoCl₂ | 0.9 | 0 | 1.2 |
| 27 | CrCl₃ | 0.3 | 0 | 0.2 |
| 28 | SnCl₂ | 0.6 | 0 | 0.8 |
| 29 | MnCl₂ | 1.2 | 0.1 | 2.2 |

### Examples 30 to 33

A catalyst which comprised 1% by weight of palladium chloride and 4% by weight of cupric chloride supported on an HY type zeolite and which was prepared by the usual immersion method and then treated at 150°C for 12 hours was packed in 7 ml reaction tubes of pyrex and the reaction tubes were heated to 150, 175, 200 nd 250 °C respectively. Then benzene, propylene and N₂/O₂ mixed gas (volume ratio =91/9) were continuously introduced into the reaction tubes at charge rates of 3.0 g/hr, 2.5 ml/min and 50 ml/min, respectively to perform reactions for 2 hours. The reaction gas was cooled by a coolant, dry ice/methanol, to liquefy and solidify the gas and then analyzed. The yields of reaction products present in the recovered reaction solution are summarized in Table 6.

**(Table 6)**

| Ex. No. | Reaction Temp. (°C ) | Yield (%) | |
|---|---|---|---|
| | | Phenol | Acetone |
| 30 | 150 | 0.7 | 4.9 |
| 31 | 175 | 1.2 | 5.3 |
| 32 | 200 | 2.0 | 7.8 |
| 33 | 250 | 3.9 | 2.2 |

### Examples 34 to 37

Reactions were performed in the same manner and under the same conditions used in Examples 30 to 33 except that catalysts which comprised palladium chloride and cupric chloride in the same amounts used in Examples 30 to 33 supported on NaY type zeolite, and which was prepared in the same manner employed therein were used. The results obtained are listed in the following Table 7.

**(Table 7)**

| Ex. No. | Reaction Temp. (°C ) | Yield (%) | |
|---|---|---|---|
| | | Phenol | Acetone |
| 34 | 150 | 0.5 | 3.2 |
| 35 | 175 | 0.9 | 4.7 |
| 36 | 200 | 1.3 | 5.6 |
| 37 | 250 | 2.1 | 1.5 |

### Example 38

A reaction was carried out under the same conditions used in Example 1 except that 0.01 g of ferrous chloride tetrahydrate, 0.01 g of ferric chloride and 0.05 g of lithium chloride were additionally added and that the reaction temperature was changed to 100 °C. As a result, phenol and acetaldehyde were formed in yields of 7.8% and 25.8% respectively. Any p-benzoquinone was not detected.

### Example 39

A reaction was carried out under the same conditions used in Example 38 which had been performed according to Example 1 except that the ferric chloride hexahydrate was not used and the resulting product was analyzed in the same manner. As a result, phenol and acetaldehyde were formed in yields of 7.4% and 19.4% respectively. Any p-benzoquinone was not detected.

### Example 40

A reaction was carried out in the same manner and under the same conditions used in Example 38 except that the ferrous chloride tetrahydrate was not used and the resulting product was analyzed in the same manner. As a result, phenol and acetaldehyde were formed in yields of 7.5% and 19.5% respectively. Any p-benzoquinone was not detected.

### Example 41

A reaction was carried out in the same manner and under the same conditions used in Example 38 except that the lithium chloride was not used and the resulting product was analyzed in the same manner. As a result, phenol and acetaldehyde were formed in yields of 7.0% and 16.6% respectively. Any p-benzoquinone was not detected.

### Example 42

A reaction was carried out in the same manner and under the same conditions used in Example 38 except that 8 kg/cm² of propylene was substituted for the ethylene used in Example 38 and the resulting product was analyzed in the same manner. As a result, phenol and acetone were formed in yields of 6.6% and 22.4% respectively. Any p-benzoquinone was not detected.

### Example 43

After carrying out the procedures used in Example 42, 8 kg/cm² of propylene was further charged to the reaction system and a reaction was further continued in the same manner used in Example 42 except that the reaction was performed at 100 °C for 3 hours. As a result, phenol and acetone were formed in yields of 10.1% and 30.4% respectively. Any p-benzoquinone was not detected.

### Example 44

After carrying out the procedures used in Example 43, 8 kg/cm² of propylene was again charged, a reaction was performed in the same manner and under the same conditions used in Example 43 and the resulting product was analyzed in the same manner. As a result, phenol and acetone were formed in yields of 13.6% and 37.9% respectively. Any p-benzoquinone was not detected.

### Example 45

A reaction was carried out in the same manner and under the same conditions used in Example 42 except that lithium chloride was not used and the resulting product was analyzed in the same manner. As a result, phenol and acetone were formed in yields of 6.2% and 20.3% respectively. Any p-benzoquinone was not detected.

### Example 46

A reaction was carried out in the same manner and under the same conditions used in Example 38 except that 4.2 g of butene-1 was substituted for the ethylene and the resulting product was analyzed in the same manner. As a result, phenol and methyl ethyl ketone were formed in yields of 6.0% and 20.2% respectively. Any p-benzoquinone was not detected.

### Example 47

A reaction and analysis of the resulting product were carried out in the same manner and under the same conditions used in Example 38 except that 4.0 g of cyclohexene was substituted for the ethylene. As a result, phenol and cyclohexanone were formed in yields of 5.8% and 8.7% respectively. Any p-benzoquinone was not detected.

### Examples 48 to 52

Reactions and analysis of the resulting products were carried out in the same manner and under the same conditions used in Example 38 except that toluene, naphthalene, anisole, p-xylene and α-methylnaphthalene (4 g each) were substituted for the benzene used in Example 38. The results obtained are summarized in Table 8. Each reaction product was obtained in a good yield as shown in Table 8.

**(Table 8)**

| Ex. No. | Aromatic Compound Used | Yield (%) | |
|---|---|---|---|
| | | Hydroxy Compound | CH₃CHO |
| 48 | toluene | 8.2 | 24.9 |
| 49 | naphthalene | 6.1 | 22.7 |
| 50 | anisole | 7.2 | 24.2 |
| 51 | p-xylene | 9.2 | 25.6 |
| 52 | α-methylnaphthalene | 8.9 | 26.3 |

### Example 53

A reaction was carried out in the same manner and under the same conditions used in Example 38 except that 100 kg/cm² of a mixed gas comprising 9% of oxygen and 91% of nitrogen was substituted for the oxygen used in Example 38. As a result, phenol and acetaldehyde were formed in yields of 7.2% and 20.2% respectively. Any p-benzoquinone was not detected.

### Example 54

A reaction was carried out under the same conditions used in Example 53 except that the reaction time was changed to one hour. As a result, phenol and acetaldehyde were formed in yields of 5.4% and 17.7% respectively. Any p-benzoquinone was not detected.

### Example 55

A reaction was carried out under the same conditions used in Example 54 except that 0.1 N sulfuric acid was substituted for the 0.1 N hydrochloric acid. As a result, phenol and acetaldehyde were formed in yields of 4.8% and 18.3% respectively. Any p-benzoquinone was not detected.

### Examples 56 to 57

Reactions were carried out under the same conditions used in Example 54 except that sodium chloride and cesium chloride (0.05 g each), respectively were substituted for the lithium chloride. The results obtained are listed in Table 9. In each reaction, any p-benzoquinone was not detected.

**(Table 9)**

| Ex. No. | Metal Compound Used | Phenol(%) | Acetaldehyde (%) |
|---|---|---|---|
| 56 | NaCl | 5.9 | 20.8 |
| 57 | CsCl | 4.7 | 16.5 |

### Example 58

A reaction was carried out under the same conditions used in Example 55 except that 0.05 g of copper sulfate was additionally added. As a result, phenol and acetaldehyde were formed in yields of 5.1% and 18.5% respectively. Any p-benzoquinone was not detected.

### Example 59

A reaction was carried out under the same conditions used in Example 58 except that the reaction temperature was changed to 150 °C. As a result, phenol, acetaldehyde and p-benzoquinone were formed in yields of 7.3%, 21.8% and 0.2% respectively..

### Example 60

A reaction was carried out under the same conditions used in Example 59 except that the reaction temperature was changed to 180 °C and that 0.1 N acetic acid was substituted for the sulfuric acid. As a result, phenol and acetaldehyde were formed in yields of 7.0% and 19.1% respectively. Any p-benzoquinone was not detected.

### Examples 61 to 64

A catalyst which comprised 1% by weight of palladium chloride, 4% by weight of cupric chloride, 0.5% by weight of ferrous chloride tetrahydrate, 0.5% by weight of ferric chloride hexahydrate and 1% by weight of lithium chloride supported on an HY type zeolite and which was prepared by the usual immersion method and then treated at 150°C for 12 hours was packed in 7 ml reaction tubes of pyrex and the reaction tubes were heated to 150, 175, 200 nd 250 °C respectively. Then benzene, water, propylene and N₂/O₂ mixed gas (volume ratio =91/9) were continuously introduced into the reaction tubes at charge rates of 3.0 g/hr, 3.9 g/hr, 2.5 ml/min and 50 ml/min, respectively to perform reactions for 2 hours. Each reaction gas was cooled by a coolant, dry ice/methanol, to liquefy the gas and then analyzed. The yields of reaction products present in the recovered reaction solution are summarized in Table 10.

**(Table 10)**

| Ex. No. | Reaction Temp. (°C ) | Yield (%) | |
|---|---|---|---|
| | | Phenol | Acetone |
| 61 | 150 | 1.3 | 8.6 |
| 62 | 175 | 1.9 | 9.2 |
| 63 | 200 | 2.7 | 11.9 |
| 64 | 250 | 4.6 | 6.1 |

### Examples 65 to 68

Reactions were performed in the same manner and under the same conditions used in Examples 22 to 25 except that catalysts which comprised the same amounts of palladium chloride, cupric chloride, ferrous chloride tetrahydrate, ferric chloride as hexahydrate and lithium chloride as used in Examples 61 to 64 supported on an NaY type zeolite and which were prepared by the same manner used therein were used. The results obtained are listed in the following Table 11.

**(Table 11)**

| Ex. No. | Reaction Temp. (°C ) | Yield (%) | |
|---|---|---|---|
| | | Phenol | Acetone |
| 65 | 150 | 1.1 | 6.7 |
| 66 | 175 | 1.6 | 8.4 |
| 67 | 200 | 2.3 | 9.8 |
| 68 | 250 | 3.1 | 4.2 |

### [Effects of the Invention]

(1) Aromatic hydroxy compounds and carbonyl compounds can be simultaneously produced.
(2) Aromatic compounds are directly oxidized to efficiently give aromatic hydroxy compounds and the yields of these aromatic hydroxy compounds can be improved through the addition of olefins.
(3) Carbonyl compounds such as aldehydes and ketones can be produced in high selectivity and efficiency.
(4) Phenol can be produced by direct oxidation under mild conditions such as a temperature and a pressure lower than those used in conventional methods.
(5) Phenol, acetaldehyde and acetone which are industrially important can be produced highly efficiently from the viewpoint of safety and process control as well as from the economical standpoint.

Thus, the present invention can provide a novel method for simultaneously preparing aromatic hydroxy compounds such as phenol and carbonyl compounds such as acetone and acetaldehyde which are industrially quite excellent.

### (Industrial Applicability)

According to the method of the present invention, aromatic hydroxy compounds and carbonyl compounds can be simultaneously produced, the aromatic hydroxy compounds can be prepared through direct oxidation of aromatic compounds under mild conditions and the yield of the aromatic hydroxy compound can further be improved by the addition of olefins. Accordingly, the present invention makes it possible to simultaneously prepare phenol, acetone and acetaldehyde, which are industrially important materials, highly efficiently from the viewpoint of safety and process control as well as from the economical standpoint.

## Claims

1. A method for simultaneously preparing a carbonyl compound and an aromatic hydroxy compound comprising reacting an olefin and an aromatic compound with an oxidizing agent in the presence of a catalyst which comprises at least one metal of the platinum group or compound of the platinum group metal and at least one metal capable of forming compounds thereof in which the metal has at least two kinds of valencies or compound thereof.

2. The method of claim 1 wherein the reaction system includes further at least one member selected from the group consisting of metals of the iron group, compounds of metals of the iron group, alkali metals and compounds of alkali metals.

3. The method of claim 1 wherein water is present in the reaction system.

4. The method of claim 1 wherein the reaction is carried out in a liquid phase containing water.

5. The method of claim 1 wherein the aromatic compound is a 6-membered hydrocarbon compound, a 6-membered carbon ring condensed compound or a heteroaromatic compound.

6. The method of claim 1 wherein the olefin is selected from aliphatic, alicyclic or aromatic olefins each having at least one carbon-carbon double bond.

7. The method of claim 1 wherein the metal of the platinum group is palladium or ruthenium.

8. The method of claim 1 wherein the metal capable of forming compounds thereof in which the metal has at least two kinds of valencies is iron, nickel, cobalt, copper, silver or gold.

9. A method according to claim 1 for the simultaneous preparation of phenol and at least one of aldehyde and ketone the method comprising reacting, in a liquid phase, olefins and benzene with an oxidizing agent in the presence of copper ions and palladium as catalyst.

10. A method according to claim 9 wherein the reaction system additionally includes at least one member selected from iron ions and lithium ions.

11. The method of claim 1 wherein the oxidizing agent is a substance capable of supplying molecular oxygen, oxygen atoms, oxygen ions or oxygen radicals to the reaction system.

12. The method of claim 11 wherein the oxidizing agent is molecular oxygen or that diluted with an inert substance.

13. The method of claim 1 wherein the reaction system is in an acidic condition.

14. The method of claim 1 wherein the olefins are additionally and intermittently introduced into the reaction system.

## Patentansprüche

1. Verfahren zur gleichzeitigen Herstellung einer Carbonylverbindung und einer aromatischen Hydroxyverbindung, umfassend das Umsetzen eines Olefins und einer aromatischen Verbindung mit einem Oxidationsmittel in Gegenwart eines Katalysators, der zumindest ein Platingruppenmetall oder eine Verbindung des Platingruppenmetalls und zumindest ein Metall, das fähig ist, Verbindungen davon zu bilden, worin das Metall zumindest zwei verschiedene Wertigkeiten aufweist, oder eine Verbindung davon umfaßt.

2. Verfahren nach Anspruch 1, worin das Reaktionssystem weiters zumindest einen Bestandteil umfaßt, der aus der Gruppe ausgewählt wird, die aus Metallen der Eisengruppe, Verbindungen von Metallen der Eisengruppe, Alkalimetallen und Alkalimetallverbindungen besteht.

3. Verfahren nach Anspruch 1, worin im Reaktionssystem Wasser vorhanden ist.

4. Verfahren nach Anspruch 1, worin die Reaktion in einer Wasser enthaltenden, flüssigen Phase durchgeführt wird.

5. Verfahren nach Anspruch 1, worin die aromatische Verbindung eine 6-gliedrige Kohlenwasserstoff-Verbindung, eine kondensierte Verbindung mit 6-gliedrigem Kohlenstoffring oder eine heteroaromatische Verbindung ist.

6. Verfahren nach Anspruch 1, worin das Olefin aus aliphatischen, alizyklischen oder aromatischen Olefinen, die jeweils zumindest eine Kohlenstoff-Kohlenstoff-Doppelbindung aufweisen, ausgewählt wird.

7. Verfahren nach Anspruch 1, worin das Platingruppenmetall Palladium oder Ruthenium ist.

8. Verfahren nach Anspruch 1, worin das Metall, das in der Lage ist, Verbindungen davon zu bilden, worin das Metall zumindest zwei verschiedene Wertigkeiten aufweist, Eisen, Nickel, Kobalt, Kupfer, Silber oder Gold ist.

9. Verfahren nach Anspruch 1 zur gleichzeitigen Herstellung von Phenol und zumindest einem von Aldehyd und Keton, wobei das Verfahren das Umsetzen von Olefinen und Benzol mit einem Oxidationsmittel in einer flüssigen Phase in Gegenwart von Kupferionen und Palladium als Katalysator umfaßt.

10. Verfahren nach Anspruch 9, worin das Reaktionssystem zusätzlich zumindest einen Bestandteil umfaßt, der aus Eisenionen und Lithiumionen ausgewählt wird.

11. Verfahren nach Anspruch 1, worin das Oxidationsmittel eine Substanz ist, die in der Lage ist, dem Reaktionssystem molekularen Sauerstoff, Sauerstoffatome, Sauerstoffionen oder Sauerstoffradikale zuzuführen.

12. Verfahren nach Anspruch 11, worin das Oxidationsmittel molekularer Sauerstoff oder derselbe verdünnt mit einer inerten Substanz ist.

13. Verfahren nach Anspruch 1, worin das Reaktionssystem in einem sauren Zustand vorliegt.

14. Verfahren nach Anspruch 1, worin die Olefine zusätzlich und intermittierend ins Reaktionssystem eingebracht werden.

## Revendications

1. Méthode de préparation simultanée d'un composé de carbonyle et d'un composé hydroxy aromatique comprenant la réaction d'une oléfine et d'un composé aromatique avec un agent oxydant en présence d'un catalyseur qui comprend au moins un métal du groupe platine ou un composé du métal du groupe platine et au moins un métal capable de former ses composés où le métal a au moins deux sortes de valences ou son composé.

2. Méthode de la revendication 1 où le système de réaction comprend de plus au moins un membre sélectionné dans le groupe consistant en métaux du groupe fer, composés des métaux du groupe fer, métaux alcalins et composés des métaux alcalins.

3. Méthode de la revendication 1 où de l'eau est présente dans le système de réaction.

4. Méthode de la revendication 1 où la réaction est effectuée dans une phase liquide contenant de l'eau.

5. Méthode de la revendication 1 où le composé aromatique est un composé d'hydrocarbure à 6 membres, un composé condensé de carbone à 6 membres ou un composé hétéroaromatique.

6. Méthode de la revendication 1 où l'oléfine est sélectionnée parmi des oléfines aliphatiques, alicycliques ou aromatiques, chacune ayant au moins une double liaison carbone-carbone.

7. Méthode de la revendication 1 où le métal du groupe platine est le palladium ou le ruthénium.

8. Méthode de la revendication 1 où le métal capable de former ses composés où le métal a au moins deux sortes de valences est le fer, le nickel, le cobalt, le cuivre, l'argent ou l'or.

9. Méthode selon la revendication 1 pour la préparation simultanée de phénol et d'au moins un aldéhyde et une cétone, la méthode comprenant la réaction, dans une phase liquide, d'oléfines et de benzène avec un agent oxydant en présence d'ions de cuivre et de palladium comme catalyseur.

10. Méthode selon la revendication 9 où le système de réaction contient additionnellement au moins un membre sélectionné parmi les ions de fer et les ions de lithium.

11. Méthode de la revendication 1 où l'agent oxydant est une substance capable de fournir de l'oxygène moléculaire, des atomes d'oxygène, des ions d'oxygène ou des radicaux d'oxygène, au système de la réaction.

12. Méthode de la revendication 11 où l'agent oxydant est de l'oxygène moléculaire ou bien celui dilué avec une substance inerte.

13. Méthode de la revendication 1 où le système de réaction est en une condition acide.

14. Méthode de la revendication 1 où les oléfines sont introduites additionnellement et par intermittence dans le système réactionnel.
